# EUROPEAN PATENT SPECIFICATION

(11) **EP 3 317 673 B1**
(45) Date of publication and mention of the grant of the patent: **26.05.2021**
(21) Application number: 16733462.2
(22) Date of filing: 28.06.2016
(51) Int. Cl.: G01N 33/68, G01N 33/92

(54) **NEW DIAGNOSTIC TOOLS FOR CHARCOT-MARIE-TOOTH DISEASE**
NEUE DIAGNOSEINSTRUMENTE FÜR MORBUS CHARCOT-MARIE-TOOTH
NOUVEAUX OUTILS DE DIAGNOSTIC POUR LA MALADIE DE CHARCOT-MARIE-TOOTH

(30) Priority: 30.06.2015 US 201514755466
(43) Date of publication of application: 09.05.2018
(73) Proprietor: Pharnext, 92130 Issy-les-Moulineaux (FR)
(72) Inventor: COHEN, Daniel, 92210 Saint-Cloud (FR); CHUMAKOV, Ilya, 77000 Vaux-le-Pénil (FR); NABIROCHKIN, Serguei, 92290 Châtenay-Malabry (FR)
(74) Representative: Cabinet Becker et Associés
(86) International application number: PCT/EP2016/064918
(87) International publication number: WO 2017/001360

(56) References cited:
- WO-A1-2011/061304
- WO-A2-2010/004283
- ILYA CHUMAKOV ET AL: "Polytherapy with a combination of three repurposed drugs (PXT3003) down-regulates Pmp22 over-expression and improves myelination, axonal and functional parameters in models of CMT1A neuropathy", ORPHANET JOURNAL OF RARE DISEASES, BIOMED CENTRAL LTD, LO, vol. 9, no. 1, 10 December 2014 (2014-12-10), page 201, XP021207652, ISSN: 1750-1172, DOI: 10.1186/S13023-014-0201-X
- MARGARET J KOVACH ET AL: "A Unique Point Mutation in the PMP22 Gene Is Associated with Charcot- Marie-Tooth Disease and Deafness", AM. J. HUM. GENET, vol. 64, 1 January 1999 (1999-01-01), pages 1580-1593, XP055348979,
- J. BERCIANO ET AL: "Guía diagnóstica en el paciente con enfermedad de Charcot-Marie-Tooth", NEUROLOGÍA, vol. 27, no. 3, 1 April 2012 (2012-04-01), pages 169-178, XP055348980, ES ISSN: 0213-4853, DOI: 10.1016/j.nrl.2011.04.015

## Description

The present invention relates generally to the field of medicine. The present invention relates in particular to methods of detecting predisposition to or diagnosis and/or prognosis of Charcot-Marie-Tooth (CMT) and related disorders. More specifically, the invention relates to development, validation and application of new biomarkers, which can be used for detecting the presence or risk of CMT disease and related disorders. In particular, the present invention relates to metabolite, lipid, carbohydrate and proteinaceous biomarkers that can be measured in biological body fluids and easily available extracts of biopsies, which can be used to aid in the detection, prediction of drug treatment and follow up of this treatment of neurodegenerative disorders, including CMT disease. The present invention also relates to methods for identification of CMT disease sub-types, assessing the responsiveness to the treatments and the efficacy of treatments in subjects having CMT or a related disorder.

Charcot-Marie-Tooth disease ("CMT") is an orphan genetic peripheral polyneuropathy. Affecting approximately 1 in 2,500 individuals, this disease is the most common inherited disorder of the peripheral nervous system. Its onset typically occurs during the first or second decade of life, although it may be detected in infancy. Course of disease is chronic with gradual neuromuscular degeneration. The disease is invalidating with cases of accompanying neurological pain and extreme muscular disability. CMT is one of the best studied genetic pathologies with approximately 30,000 cases in France. While a majority of CMT patients harbour a duplication of a chromosome 17 fragment containing a myelin gene: PMP22 (form CMT1A), more than two dozens of genes have been implicated in different forms of CMT. Accordingly, although monogenic in origin, this pathology manifests clinical heterogeneity due to possible modulator genes. The genes mutated in CMT patients are clustering around tightly connected molecular pathways affecting differentiation of Schwann cells or neurons or changing interplay of these cells in peripheral nerves.

At present, the diagnostic of CMT disease is based on clinical criteria and electrophysiology data that distinguish only few subtypes of this disease. More precise classification relies on mutation's analysis in relevant genes if known.

The multiple mutations leading to CMT disease occur in more than 25 different genes. They are not identified for all cases of CMT disease and cannot be exhaustingly classified by genetic typing (Suter & Scherer, 2003; Berger *et al.,* 2006; Niemann *et al.,* 2006; Nave *et al.,* 2007). Moreover, clinical heterogeneity does occur and not only is important for clinical characterization but provides further implication of specific management/treatment for functionally different forms (Sereda *et al.,* 2003; Passage *et al.,* 2004; Sahenk *et al.,* 2005; Young *et al.,* 2008).

For the moment, no drug treatment exists for this disease but some clinical management procedures have been described (Grandis & Shy, 2005; Kapur *et al.,* 2007; Weiner *et al.,* 2008) and clinical trials with ascorbic acid for the treatment of CMT1A form of this disease are under way (Burns *et al.,* 2009).

A specific tool to measure the stage of the disease is CMT Neuropathy Score (CMTNS, Shy et al. 2005). CMTNS is used to measure patient disability in CMT patients and as an outcome measure in treatment trials. It is a composite score gathering the results of symptoms, signs, and neurophysiological tests. Patients are classified as mild (CMTNS ≤ 10), moderate (CMTNS 11 - 20), or severe (CMTNS > 20) depending of their performances assessed in nine tests.

The Overall Neuropathy Limitations Scale (ONLS), though not specifically designed for CMT, is used to record serial changes in limitations in a clinical environment and as an outcome measure in clinical trials for patients suffering from neuropathies. It measures limitations in the everyday activities of the upper and lower limbs (Graham and Hughes, 2006).

The progression of this disease measured by CMTNS or ONLS is rather slow and necessitates long clinical trials with hundreds of patients.

The protein and RNA levels of PMP22 have been recently proposed as biological markers to follow up such trials pharmacodynamically as a substitute for CMTNS endpoint in a case of CMT1A (Li *et al.,* 2005 ; Meyer zu Horste *et al.,* 2007). Still, such analysis is tedious and requires invasive procedure. Moreover, expression of PMP22 in such biopsies is not correlated with severity of disease (Katona *et al.,* 2009).

Patrocolo *et al.,* 2009 reports elevated total cholesterol and triglycerides levels in a patient with Autosomal Dominant Hereditary Motor Sensory Neuropathy with Proximal Dominant Involvement (HMSN-P).

Yao *et al.,* 1978 studies the distribution of specific fractions of cholesteryl esters in patients having hereditary neuropathies. This document provides no information regarding free cholesterol or LDL cholesterol levels.

Swartz et *al.,* 1988 concerns immunogenicity of cholesterol and production of monoclonal IgM complement-fixing antibodies to cholesterol.

Niebroj-Dobosz et *al.,* 1976 concerns patterns of different lipid fractions in neuropathic patients (such as total lipids, total phospholipids, free fatty acids or cholesterol esters). The authors conclude that there is no correlation between the type of lipid pattern changes and the clinical syndrome.

WO2011/061304 concerns a method for detecting predisposition to or diagnosis and/or prognosis of Charcot-Marie-Tooth disease (CMT) and shows the quantification of several biomarkers, such as alanine or tryptophan in a CMT1A transgenic rat model and in WT rats. Chumakov et *al.,* 2014 relates to the use of a combination of baclofen, naltrexone and sorbitol for down-regulating the Pmp22 over-expression in patients suffering from CMT1A disease.

The availability of easily detectable biological markers would permit rapid diagnosis of functionally relevant forms of CMT and related diseases, clinical testing of efficacy of new medications and monitoring the individual response of patients to drug treatment and disease management.

### SUMMARY OF INVENTION

The purpose of the present invention is to provide novel methods for detecting predisposition to, or diagnosis and/or prognosis of CMT disease and related disorders, as well as for assessing the responsiveness to the treatments and/or the efficacy of treatments in subjects having CMT or a related disorder.

The object of this invention relates to a method as defined in the claims.

Also described herein is a method for the diagnosis of CMT and CMT related diseases. Also described herein are methods for aiding in the diagnosis and sub-classification of neurological disorders, or in patient stratification step in clinical trials, including CMT and CMT related diseases, by quantifying the amount of lipids, amino acids, steroid hormones, carbohydrates, metals, arachidonic acid metabolites, biogenic amines, nucleosides, nucleotides, small peptides and proteins in a biological fluid sample of the subject, such as a cerebrospinal fluid, serum, saliva, urine, etc. and comparing the measured amount with a reference value for the biomarker. These methods can also be applied to quantification of biomarkers in extracts of biopsies including skin biopsy. The information thus obtained may be used to aid in the diagnosis, to diagnose the disease, or to predict potential drug response in the individual. The biomarkers are differentially present in subjects having a neurological disease, including CMT and CMT related diseases, versus subjects free of the disease.

Also described herein is a method of diagnosing or assessing the likelihood that a patient is afflicted with a neurological disease, including CMT, preferably CMT1A, and CMT related diseases, the method comprising measuring a level of complex combination biomarkers of the present invention.

Described herein is an *in vitro* method for detecting the presence or risk of CMT disease in a mammal, or for aiding in the diagnosis, prognosis or sub-classification of CMT disease, or in patient stratification step in clinical trials, the method comprising determining the (relative) amount or the presence, absence or alteration of a target biomarker in a fluid biological sample from the subject, wherein said amount or alteration is indicative of the presence, risk, progression or severity of said disease, and wherein said biomarker is selected from lipids, amino acids, steroid hormones, metals, metabolites of arachidonic acid, biogenic amines, carbohydrates, peptides, nucleosides and nucleotides.

Also described herein is an *in vitro* method for assessing efficacy of a treatment against CMT in a mammal, the method comprising determining in a fluid biological sample from the subject, during the treatment, the (relative) amount or the presence, absence or alteration of a target biomarker selected from lipids, amino acids, steroid hormones, metals, metabolites of arachidonic acid, biogenic amines, carbohydrates, peptides, nucleosides and nucleotides, and comparing said amount or alteration to a level of said biomarker determined before treatment or at an earlier stage of treatment in said mammal, wherein a deviation is indicative of the efficacy of the treatment.

The methods described herein may use one or more target biomarker(s). In a preferred embodiment, said target biomarker(s) are selected from cholesterol, alanine, α-aminobutyric acid, citrulline, cystine, glutamine, hydroxyproline, lysine, methionine, proline, threonine, tryptophan, tyrosine, T4 thyroid hormone, testosterone, iron, LTB4, adrenaline, dopamine and serotonin, or combinations thereof.

In another embodiment, said one or more biomarkers are used in conjunction with at least one additional diagnostic test or marker for CMT, selected preferably from nucleic acids, proteinous, physiological, neurophysiological, genetic, behavioral, electrophysiological, clinical and phenotypical test or marker.

Also described herein is a use of one or more biomarker(s) disclosed herein in a method of detecting predisposition to or diagnosis and/or prognosis of CMT disease in a mammalian subject.

A further disclosure of the invention is to provide an *in vitro* method for predicting the responsiveness to a treatment of CMT disease of an individual suffering from CMT, the method comprising:
i) determining the free cholesterol level in a biological sample from said individual, and
ii) predicting the responsiveness of said individual to said treatment by comparing the free cholesterol level obtained in i) to a reference value of a responder or non-responder group.

Also described herein is an *in vitro* method for determining the progression of CMT disease in an individual having CMT, the method comprising:
i) determining the level of alanine or tryptophan, or both, in a biological sample from said individual, and
ii) comparing the level of alanine or tryptophan, or both, obtained in i) to level(s) of alanine and/or tryptophan, respectively, determined previously in the same individual,
wherein a change in the level of alanine and/or tryptophan is indicative of the progression of CMT disease in said individual.

Also described herein is a method of partitioning a group of patients suffering from CMT disease comprising determining the levels of alanine or tryptophan, or both, in a biological sample from said patients, wherein said level(s) is/are used to partition said group of patients as a function of the severity of the disease.

### BRIEF DESCRIPTION OF THE FIGURE

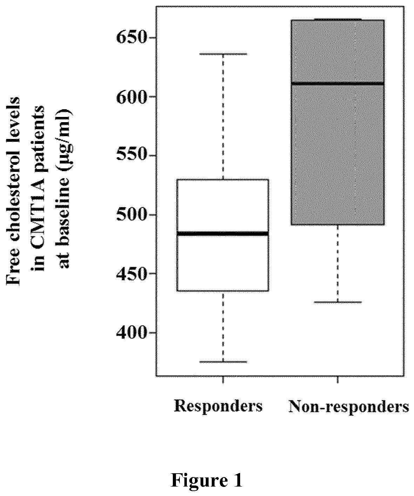

**Figure 1****:** Free cholesterol levels in CMT1A patients at baseline. Before the beginning of treatment with a mix of baclofen, naltrexone and sorbitol, free cholesterol levels were found significantly lower in patients who turned out to respond to the treatment, i.e., whom condition was stabilized or improved (responders, white box) in comparison with those whom turned out to not respond to the treatment, i.e., whom condition worsened after one year of treatment (non-responders, grey box). (p<0.034, Welch t-test, free cholesterol levels of responders significantly different from non-responders).

### DETAILED DESCRIPTION OF THE INVENTION

The present invention provides new diagnostic methods and tools for CMT and related disorders.

Within the context of this invention, CMT includes CMT1A, CMT1B, CMT1C, CMT1D, CMT1X, CMT2A, CMT2B, CMT2D, CMT2E CMT2F, CMT2I, CMT2J, CMT2-P0, CMT2K, CMT4A, CMT4B1, CMT4B2, CMT4C, CMT4D, CMT4F, CMT4, AR-CMT2A, CMT4J or other forms of Charcot-Marie-Tooth disease. In the most preferred embodiment, CMT is CMT1A.

Within the context of the present invention, the term "CMT related disorder" designates other diseases associated with neurological symptoms. The term "CMT related disorder" more particularly includes Alzheimer's disease (AD), senile dementia of AD type (SDAT), Parkinson's disease, Lewis body dementia, vascular dementia, autism, mild cognitive impairment (MCI), age-associated memory impairment (AAMI) and problem associated with ageing, post-encephalitic Parkinsonism, schizophrenia, depression, bipolar disease and other mood disorders, Huntington's disease, motor neurone diseases including amyotrophic lateral sclerosis (ALS), multiple sclerosis, idiopathic neuropathies, diabetic neuropathy, toxic neuropathies including neuropathy induced by drug treatments, neuropathies provoked by HIV, radiation, heavy metals and vitamin deficiency states, prion-based neurodegeneration, including Creutzfeld-Jakob disease (CJD), bovine spongiform encephalopathy (BSE), GSS, FFI, Kuru and Alper's syndrome.

The purpose of the present invention is to provide new body fluid biomarkers for diagnosing or monitoring CMT and related disorders, and for assessing the responsiveness of subjects or the efficacy of therapeutic treatments in subjects having CMT or a related disorder. Thus, according to a preferred embodiment, the method of the invention comprises the detection of the presence or absence or (relative) quantity of metabolites in body fluids.

Disclosed herein is detecting (*in vitro* or *ex vivo*) the presence of or risk of developing CMT or a related disorder in a mammal, comprising the determination of the presence, in a biological sample of the mammal of an alteration in one or more selected body fluid biomarkers.
a method for detecting (*in vitro* or *ex vivo*) the presence of or risk of developing CMT or a related disorder in a mammal, comprising the determination of the presence, in a biological sample of the mammal, of an alteration of the level in one or more markers, the presence of such an alteration being indicative of the presence of or risk of developing CMT in said mammal.

Also disclosed herein is an *in vitro* method for detecting the presence or risk of CMT disease in a mammal, or for aiding in the diagnosis, prognosis or sub-classification of CMT disease, or aiding in patient stratification step in clinical trials, the method comprising determining the (relative) amount or the presence, absence or alteration of a target biomarker in a fluid biological sample from the subject, wherein said amount or alteration is indicative of the presence, risk, progression or severity of said disease, and wherein said biomarker is selected from lipids, amino acids, steroid hormones, metals, metabolites of arachidonic acid, biogenic amines, carbohydrates, peptides, nucleosides and nucleotides.

Within the context of the present invention, the term "alteration" of a target biomarker may designate an increase or a decrease of the target biomarker quantity in a fluid biological sample from the subject, in comparison with a control sample or reference value. Typically, the term "decrease" in relation to a biomarker level, designates a reduction of the concentration or level of the biomarker in a biological sample from the subject of at least 5% or 10% or 15% in comparison with a control sample or reference or mean value. Decreases may be more substantial, such as a reduction by at least 20% or 30% or 40% or even more. Similarly, the term "increase" in relation to the biomarker level, designates an augmentation of the concentration or level of the biomarker in a biological sample from the subject of at least 5% or 10% or 15% in comparison with a control sample or reference or mean value. Increases may be more substantial, such as increases by at least 20% or 30% or 40%, or even more.

Preferred types of alterations are disclosed below for each biomarker in Table A. This table indicates, for each biomarker, whether an increase or a decrease is indicative of CMT in human subjects. A distinction between male and females patients is also provided.

**Table A : Increase (+) or decrease (-) of a biomarker concentration in CMT patients**

| | ***total*** | ***female*** | ***male*** |
|---|---|---|---|
| **Adrenaline** | | - | |
| **Alanine** | - | - | |
| **Alpha Amino Butyric acid** | | - | |
| **Citrulline** | | | - |
| **Cystine** | - | | |
| **Dopamine** | + | | |
| **Free cholesterol** | - | - | - |
| **Glutamine** | - | - | |
| **Hydroxyproline** | - | - | |
| **Iron** | + | | |
| **LDL cholesterol** | | | - |
| **LTB4** | | - | |
| **Lysine** | | - | |
| **Methionine** | | | + |
| **Proline** | | | + |
| **Serotonin** | + | | |
| **T4** | - | | - |
| **Testosterone** | | | + |
| **Threonine** | - | | |
| **Tryptophane** | + | | + |
| **Tyrosine** | | - | |

Specific examples of alterations of each target biomarker(s) are shown in Tables 1-4 of the experimental part.

Also disclosed herein is qualifying and sub-classifying a CMT disease, for example CMT1A, CMT1B, CMT1C, CMT1D, CMT1X, CMT2A, CMT2B, CMT2D, CMT2E CMT2F, CMT2I, CMT2J, CMT2-P0, CMT2K, CMT4A, CMT4B1, CMT4B2, CMT4C, CMT4D, CMT4F, CMT4, AR-CMT2A, CMT4J or other forms of Charcot-Marie-Tooth disease or CMT related disorders in a subject, comprising measuring sets of complex biomarkers of the present invention.

Methods described herein further comprise the step of managing the individual treatment. For example, if measurement of the set of biomarkers correlates with the presence of clinical sub-type CMT disease, then managing treatment comprises administering a matched drug or drug combination to slow or revert the progression of the disease. Further measurements can be compared to the previous measurements, or the standard to monitor the progression of the disease.

In another aspect of the invention, the method further comprises measuring the biomarker after treatment has begun, to monitor the progression of the disease.

In another embodiment, the method of the present invention comprises monitoring the progression of a CMT, preferably CMT1A, and measuring a level of sets of biomarkers of the present invention.

Also described herein is a method to evaluate or follow the response to a treatment for CMT in a subject, the method comprising a step of measuring the level of one or more markers, the presence of such an alteration before and/or during the treatment, and a comparison of the level thus measured with that measured at a former stage of the treatment or before treatment.

Also described herein is a method to evaluate or follow the response to a treatment of CMT in a subject, the method comprising a step of measuring the amount of one or more selected body fluid biomarkers before and/or during the treatment, and a comparison of the amount thus measured with that measured at a former stage of the treatment or before treatment.

The level of the biomarker(s), measured, as described herein, is correlated with neurological disease, preferably CMT disease. In preferred embodiments, this may be accomplished by comparing the measured amount to a reference value for the biomarker(s). The reference value can be obtained by measuring an amount of the biomarker(s) in age-matched control subjects that are not affected by the disease, or that are free of the disease.

Also described herein is an *in vitro* method for assessing efficacy of a treatment against CMT in a mammal, the method comprising determining in a fluid biological sample from the subject, during the treatment, the (relative) amount or the presence, absence or alteration of a target biomarker selected from lipids, amino acids, steroid hormones, metals, metabolites of arachidonic acid, biogenic amines, carbohydrates, peptides, nucleosides and nucleotides, and comparing said amount or alteration to a level of said biomarker determined before treatment or at an earlier stage of treatment in said mammal, wherein a deviation is indicative of the efficacy of the treatment.

Also disclosed herein is monitoring the efficacy of a treatment method of a CMT, comprising measuring a level of complex set of biomarkers of the present invention. The efficacy of treatment is measured by monitoring levels of the biomarker in the subject compared to a reference, and/or compared to other previous tests of the subject or to an earlier stage of treatment/disease in the subject.

Also disclosed herein is an improvement in methods of treating CMT or related disorders, the improvement consisting in measuring the level of expression of one or, preferably, several biomarkers before and/or during the treatment. The measurement of the level of biomarker expression, makes it possible to adapt the treatment according to the evolution of pathology and/or efficacy of the treatment.

In a preferred disclosure, diagnosing or monitoring CMT and related disorders, comprises the determination of the quantity (or of the presence or of the absence), in a biological sample of the mammal, of said body fluid biomarker(s) selected from lipids, amino acids, steroid hormones, carbohydrates, metals, metabolites of arachidonic acid, biogenic amines, nucleosides and nucleotides, small peptides and proteins.

In a further preferred disclosure, the method of the invention comprises the determination of the quantity (or of the presence or of the absence), in a biological fluid sample of the mammal, of one or more body fluid biomarkers, wherein said body fluid biomarkers are selected from:
- lipids, preferably cholesterol and its metabolites, including dehydroepiandrosterone (DHEA), and including more preferably free cholesterol or LDL cholesterol, or their amount in regard of total cholesterol,
- amino acids or their derivatives, preferably including alanine, α amino butyric acid, citrulline, cystine, glutamine, hydroxyproline, lysine, methionine, proline, threonine, tryptophan and tyrosine, and/or arginine, asparagines, aspartic acid, glutamic acid, glycine, histidine, 1-methyl histidine, isoleucine, leucine, ornithine, phenylalanine, serine, taurine and valine,
- steroid hormones and their precursors or derivatives, preferably including T3 and T4 thyroid hormones, testosterone, 5α-dihydroprogesterone, allopregnanolone and corticosterone,
- metals, preferably iron and zinc,
- metabolites of arachidonic acid, preferably including leukotrienes (e.g.,LTB4/5), and prostaglandins PGE2, prostacyclins PGI2 and tromboxanesTXA2 and TXB2,
- biogenic amines, preferably including adrenaline, dopamine and serotonin,
- carbohydrates, preferably sorbitol,
- nucleotides, preferably 3'-5'-cyclic adenosine monophosphate (cAMP), and
- any combination thereof.

More preferably, said body fluid biomarkers are selected from:
- lipids, preferably cholesterol and its metabolites, including free cholesterol or LDL cholesterol, or their amount in regard of total cholesterol,
- amino acids or their derivatives, preferably including alanine, α amino butyric acid, citrulline, cystine, glutamine, hydroxyproline, lysine, methionine, proline, threonine, tryptophan and tyrosine,
- steroid hormones, preferably including T4 thyroid hormone and testosterone,
- metals, preferably iron,
- metabolites of arachidonic acid, preferably including leukotrienes (e.g., LTB4/5),
- biogenic amines, preferably including adrenaline, dopamine and serotonin, and
- any combination thereof.

The method disclosed herein comprises the determination of the quantity (or of the presence or of the absence), in a biological fluid sample of the mammal, of one or more body fluid biomarkers, wherein said body fluid biomarkers are selected from:
- cholesterol metabolites, preferably including an ester of cholesterol, 27-hydroxycholesterol, pregnenolone, pregnenolone sulfate, and dehydroepiandrosterone sulfate (DHEAS),
- steroid hormones and their precursors or derivatives, preferably including cortisol, cortisone, aldosterone, androstanediol, androstenedione, estradiol and estrone,
- metabolites of arachidonic acid, preferably including prostaglandins PGD2 and PGF2α, 12-Hydroxyeicosatetraenoic acid (12-HETE) and lipoxins (LXA4 and LXB4),
- inositol and its derivatives, preferably including inositol monophosphates, phosphatidylinositol 3-phosphate [PI3P] and phosphatidylinositol (3,5)-bi-phosphate [PI(4,5)P2],
- sphingolipids or phospholipids or their derivatives, preferably including lysophosphatidic acid, phosphatidic acid and sphingosine-1-phosphate (SIP),
- endocannabinoids, preferably including arachidonoylethanolamine, 2-arachidonoyl glycerol, 2-arachidonyl glyceryl ether, N-arachidonoyl-dopamine and virodhamine, and
- any combinations thereof.

Also described herein is a method of the invention comprises determining in a fluid biological sample from the subject the (relative) amount or the presence, absence or alteration of a target biomarker selected from cholesterol, alanine, α-aminobutyric acid, citrulline, cystine, glutamine, hydroxyproline, lysine, methionine, proline, threonine, tryptophan, tyrosine, T4 thyroid hormone, testosterone, iron, LTB4, adrenaline, dopamine and serotonin, as well as any combinations thereof.

The biomarker disclosed herein is or comprises at least cholesterol, more preferably free cholesterol, and/or LDL cholesterol and/ or their amount in regard of total cholesterol. The term "LDL cholesterol" designates all forms of cholesterol contained in LDL, including non esterified cholesterol.

As shown in the experimental part, the inventors have surprisingly discovered that the level of free cholesterol or the level of LDL cholesterol decreases in diseased animals.

Thus, the method disclosed herein comprises determining a decrease of free cholesterol and/or LDL cholesterol and/or their amount in regard of total cholesterol, in a fluid biological sample from the subject, wherein said decrease of free cholesterol and/or LDL cholesterol and/or their amount in regard of total cholesterol, is indicative of the presence, risk, progression or severity of the disease.

The method disclosed herein comprises determining in a fluid biological sample from the subject, a decrease of the ratio of free cholesterol to total cholesterol.

In another particular disclosure, the method of the invention comprises determining a decrease of the ratio of LDL cholesterol to total cholesterol.

As indicated, the method may comprise the determination of several biomarkers, e.g., 2, 3, 4, 5 or even more. These may be determined simultaneously or sequentially in a fluid biological sample.

In a particular variant, the presence or the absence or the (relative) quantity of at least three biomarkers is determined simultaneously or sequentially in a fluid biological sample from the mammalian subject.

The method disclosed herein comprises the determination of the presence or the absence or the (relative) quantity, in a biological sample of the mammal, of at least four distinct biomarkers.

The sets of biomarkers used in methods described herein are selected from Table 5.

In a preferred disclosure, the sets of biomarkers comprise:
- free-cholesterol and alanine;
- free-cholesterol and T4 and tryptophane and hydroxyproline;
- free-cholesterol and hydroxyproline;
- free-cholesterol and T4 and tryptophane;
- free-cholesterol and T4 and serotonin;
- free-cholesterol and T4 and hydroxyproline;
- free-cholesterol and T4; or
- free-cholesterol and serotonin.

As illustrated in the examples, such sets of biomarkers are particularly efficient in predicting the presence of CMT disease. In particular, the results depicted in the examples show performances of 100% in training tests and between 78% and 100% in validation tests for these sets of biomarkers.

The level of said biomarker(s) may be determined by any methods known per se in the art, such as, without limitation, immunological methods, biochemical methods, chromatographic methods, enzymatic methods, cell based assays, *in vitro* tests, etc. Examples of suitable methods are disclosed in the experimental section. The level of biomarker(s) determined may be compared to a reference value, a control, or a mean value, wherein a deviation from said value is indicative of the presence, risk, progression or severity of CMT. The deviation should typically be superior to 5%, more preferably to 10%, even more preferably 15%.

Also described herein is a use of one or more biomarker(s) selected from cholesterol, alanine, α-aminobutyric acid, citrulline, cystine, glutamine, hydroxyproline, lysine, methionine, proline, threonine, tryptophan, tyrosine, T4 thyroid hormone, testosterone, iron, LTB4, adrenaline, dopamine and serotonin in a method of detecting predisposition to or diagnosis and/or prognosis of CMT disease in a mammalian subject.

Also, as illustrated in the experimental part, the inventors have found that within a diseased population suffering from CMT, free cholesterol levels are also predictive of the responsiveness of a patient to a treatment for CMT. More particularly, the invention shows that, in a population of CMT patients that responds to a treatment with baclofen, naltrexone and sorbitol, free cholesterol levels are significantly lower than in a population that does not respond to such treatment.

Thus, also described herein is *an vitro* method comprising identifying a patient more likely to respond to a treatment of CMT by measuring free cholesterol level in said patient. Preferably, the method comprises comparing said free cholesterol level determined in a patient to a reference value of a non-responder or responder group, wherein said comparison allows determining the likelihood that the patient is a responder or non-responder. The method described herein comprises comparing said free cholesterol level determined in a patient to a reference value of a non-responder group, wherein a significantly lower level of free cholesterol when compared to said reference value is indicative that the patient shall respond to the treatment.

As shown in the experimental part, the reference value of free cholesterol (in plasma) in a responder group is typically comprised between 446 and 520 µg/mL, more particularly between 446 and 483 µg/mL, even more particularly less than 483 µg/mL.

Also described herein is the *in vitro* method comprising determining the free cholesterol level of said individual from a plasma sample obtained from the individual, wherein a free cholesterol level in said patient comprised between 446 and 520 µg/mL, more preferably between 446 and 483 µg/mL, even more preferably below 483 µg/mL, is indicative that the patient shall respond to said treatment. In a further embodiment, the *in vitro* method comprises comparing the plasma free cholesterol level of said patient with a reference value of a responder group with similar age, sex, condition, and/or any ongoing treatment (e.g., statin treatment).

As shown in the experimental part, the reference value of free cholesterol (plasma free cholesterol) in a non-responder group is typically superior to 520 µg/mL, even more particularly superior to 578 µg/mL.
the disclosed *in vitro* method comprises determining free cholesterol level in plasma of said patient, wherein a free cholesterol level superior to 520 µg/mL, even more preferably superior to 578 µg/mL, is indicative that the patient shall not respond to said treatment.

In a particular embodiment, the method allows determining responsiveness to a treatment of CMT comprising co-administering baclofen, naltrexone and sorbitol, or salts thereof, to said patient.

In a particular embodiment, the term "significantly lower" designates a level that is at least 5% lower, more preferably at least 10% lower, even more preferably at least 15% or more lower than the reference value.

In a particular embodiment, the fluid biological sample is a blood sample of the subject, preferably a plasmatic fraction from said subject.

In a particular embodiment, the above biomarkers are used in a method of detecting predisposition to, diagnosis and/or prognosis of CMT disease, or aiding in patient stratification step in clinical trials, in conjunction with at least one additional diagnostic test or marker for CMT, selected preferably from proteinous, physiological, neurophysiological, genetic, behavioral, electrophysiological, clinical and phenotypical test or marker.

In another particular embodiment, the level of said biomarker(s) used in a method of detecting predisposition to or diagnosis and/or prognosis of CMT disease, or aiding in patient stratification step in clinical trials, is compared to a reference value wherein the deviation from said value is indicative of the presence, risk, progression or severity of CMT.

Said biomarkers used in a method described herein for aiding in patient stratification step in clinical trials or evaluating progression or severity of CMT, comprise at least tryptophan (trp) and/or alanine (ala).

As shown in the experimental part, the inventors have surprisingly discovered that the variation of trp and/or alanine level(s) in CMT patients are correlated with the severity of the disease as determined by electrophysiological, clinical tests or functional measures.

Accordingly, described herein is an *in vitro* method for determining the progression of CMT disease in an individual having CMT, the method comprising:
i) determining the level of alanine or tryptophan, or both, in a biological sample from said individual, and
ii) comparing the level of alanine or tryptophan, or both, obtained in i) to level(s) of alanine or tryptophan, respectively, determined previously in the same individual,
wherein a change in the level of alanine and/or tryptophan is indicative of the progression of CMT disease in said individual.

In a preferred embodiment, the invention resides in an *in vitro* method for assessing the evolution of CMT disease in an individual having CMT and undergoing a treatment for CMT, the method comprising:
i) determining the levels of alanine and tryptophan in a biological sample from said individual, and
ii) comparing the levels of alanine and tryptophan obtained in i) to the levels of alanine and tryptophan, determined previously in the same individual before the beginning of the treatment, or at an early stage of said treatment,
wherein the time interval between the two measures of the levels of alanine and tryptophan from steps i) and ii) is at least 2 months, preferably 3 or 4 months, and wherein a relative increase in the levels of alanine and tryptophan is indicative of an improvement or stabilization of CMT disease in said individual.

The method of the invention comprises determining an increase of trp and/or ala level(s) in a fluid biological sample from the subject in regard with previously determined level(s) in said patient, wherein said increase of trp and/or ala level(s) is indicative of an improvement of CMT disease.

Determining a level of trp and/or ala levels in a fluid biological sample from the subject is made in conjunction with at least one additional diagnostic test or marker for CMT, selected preferably from proteinous, physiological, neurophysiological, genetic, behavioral, electrophysiological, clinical and phenotypical test.

Determining the level of trp and/or ala level(s) in a fluid biological sample from the subject is made in conjunction with the determination of CMTNS and/or ONLS which are known from the skilled in the art.

Determining the level of trp and/or ala level(s) in a fluid biological sample from the sample is made in conjunction with one or more of the assessments that compose CMTNS and/or ONLS which are well known from the skilled in the art.
the method described herein comprises determining the level(s) of trp and/or ala in fluid biological samples from a group of patients wherein said level(s) is/are used to partition said group of patients as a function of the severity of the disease.

In a particular embodiment the fluid biological sample is a blood sample of the subject, preferably plasma or a plasma fraction from said subject.

As shown in the experimental section, trp and/or ala level(s) is/are surprisingly found to vary as a function of the presence of a treatment of the disease which has been found to be effective in treating CMT. Indeed in the course of a clinical trial for evaluating a mix of baclofen, naltrexone and sorbitol as a treatment for CMT disease, the Inventors found that the level(s) of trp and/or ala were significantly higher in the treated population of patients when compared to patients administered with placebo, and this as soon as 3 months after the beginning of the treatment, whereas a disease evolution on such a short period cannot be determined using neither CMTNS nor ONLS.

Described herein is a method for assessing the response or responsiveness to a treatment of CMT, the method comprising measuring an increase of the level(s) of trp and/or ala in a fluid biological sample from a subject undergoing a treatment for CMT in regard with previously determined levels in said subject before the beginning of, or at an earlier stage of said treatment, wherein said increase is indicative of a response to said treatment.

In a particular embodiment the time interval between the two measures is 2 months or more, preferably 2, 3, or 4 months.

The method for assessing the response to a treatment of CMT comprise measuring an increase of level(s) of trp and/or ala in a fluid biological sample from a subject undergoing a treatment for CMT for 3 months, in regard with previously determined levels before the beginning of said treatment.

Typically, an increase in trp and/or ala level(s) superior to 5%, more preferably to 10%, even more preferably 15% is indicative of a response to a treatment.

In an even more preferred embodiment the above method is used to assess the response to a combinatory treatment for CMT comprising the administration of baclofen, naltrexone and sorbitol.

In particular embodiment, any of the above mentioned body fluid biomarkers, or their combinations, can be used in conjunction with at least one additional diagnostic test or marker for CMT, selected preferably from nucleic acids, proteinous, physiological, neurophysiological, genetic, behavioral, electrophysiological, clinical and phenotypical test or marker.

Said proteinous biomarkers, detectable in body fluids, which can be used for diagnostic of CMT or for monitoring of progression of CMT, or for monitoring of the efficacy of CMT-relevant drugs, include NEFH neurofilament, p75/LNGFR nerve growth factor receptor, NTRK3 receptor, SCIP transcription factor, cyclin D1, lysosomal-associated membrane protein LAMP1, ATG7 autophagy related 7 homolog, proteasome activator subunits PSME1/2, PSMA1 proteasome subunit, ITGB1/4 integrins, insulin-like growth factor 1 (IGF1), insulin-like growth factor binding proteins 1/2/5 (IGFBP1/2/5), vitronectin (VTN), tenascins (TNC/R/XB), SCN10A voltage-gated sodium channel, KCNC1 potassium voltage-gated channel, aldose reductases including AKR1B1, sorbitol dehydrogenase (SORD), inositol(myo)-1(or 4)-monophosphatases IMPA1/2, ADP-ribosylation factor 6 (ARF6), calnexin (CANX), growth factors FGF2, PDGFA/B/C, VEGFA/B/C and TGFB1/2, neuregulins including NRG1, matrix metallopeptidase 2/9, tissue and urokinase plasminogen activators PLAT and PLAU, monocyte chemoattractant protein-1 (CCL2), leukemia inhibitory factor (LIF), interleukin 6, transferrin, and endogenous opioids POMC, PENK and PDYN as well as smaller peptides and other derivatives produced by metabolism of above mentioned molecules.

Additional protein biomarkers, useful for diagnostic of Charcot-Marie-Tooth disease (CMT) or for monitoring of progression of CMT, or for monitoring of efficacy of CMT-relevant drugs, can be selected from peripheral myelin protein 22 PMP22, from ciliary neurotrophic factor CNTF, fatty acid elongase ELOV16, glypican GPC3, myosins MYO1B/1G, phosphoprotein enriched in astrocytes PEA15, calcium binding proteins S100A3/4, troponins TNNT1/3 and ferritin FTH1 as well as smaller peptides and other derivatives produced by their metabolism.

Further protein biomarkers, detectable in body fluids and useful for diagnostic of Charcot-Marie-Tooth disease or for monitoring of progression of CMT, or for monitoring of efficacy of CMT-relevant drugs, can be selected from proteins or smaller peptides and their derivatives encoded by ATP1A1, FGL2, ACAT2, ACTN2, AK1, ANK3, ANXA1, APOD, CD151, CD24A, CD9, CD99, CETN2, CHN1, CLIC4, COL1A1/2, COL2A1, COL3A1, COL4A1, CRYAB, CTSC, CYB5B, CYB561, DEAF1, EMID1, EPB4.1L2, EZR, FASN, FBLN2, FDFT1, FHL1, FOS, GAPD, GATM, HBA1, HBB, IGF2, ITIH5, KIT, LGALS1, LPL, LXN, MAPK3, MFGE8, MGLL, MMP12, MRAS, MSLN, MTAP1B, NECL1, NPR3, ODF2, OGN, OLFM1, PCOLCE, PMM1, PROS1, PYGM, RAB2, RAP1GDS1, SERPINE2, SH3GL3, SIRT2, SPP1, TPM1/2, TUBA2 and UCHL1 genes.

The above groups of genes (or the corresponding proteins or ligands) represent valuable biomarkers which may be used, alone or in various combinations, to diagnose CMT or related disorders.

Also disclosed herein is a kit comprising a solid support comprising at least one capture agent attached thereto, wherein the capture agent binds or reacts with one or more component(s) of the biomarker protein complex of the present invention.

The kit described herein comprises a solid support comprising at least one capture agent attached thereto, wherein the capture agent binds or reacts with at least one biomarker selected from cholesterol, alanine, α-aminobutyric acid, citrulline, cystine, glutamine, hydroxyproline, lysine, methionine, proline, threonine, tryptophan, tyrosine, T4 thyroid hormone, testosterone, iron, LTB4, adrenaline, dopamine and serotonin. The kit described hereincomprises at least one compound binding to or reacting with at least one biomarker selected from cholesterol, alanine, α-aminobutyric acid, citrulline, cystine, glutamine, hydroxyproline, lysine, methionine, proline, threonine, tryptophan, tyrosine, T4 thyroid hormone, testosterone, iron, LTB4, adrenaline, dopamine and serotonin for the diagnostic, prognostic and/or for assessing the efficacy of a treatment or following the evolution of CMT1A disease.

The method of the invention is applicable to any biological sample of the mammal to be tested, in particular any sample comprising metabolites. Examples of such samples include blood, plasma, serum, saliva, urine, feces, tissue biopsy, etc. The sample can be obtained by any technique known per se in the art, for example by collection using e.g., non-invasive techniques, or from collections or banks of samples, etc. The sample can in addition be pretreated to facilitate the accessibility of the target molecules, for example by lysis (mechanical, chemical, enzymatic, etc.), purification, centrifugation, separation, etc.

The invention is applicable to any mammal, preferably to a human.

Further aspects and advantages of this invention will be disclosed in the following experimental section, which shall be considered as illustrative only.

### EXAMPLES

### I. Identification of New Markers and Quantitation of biomarkers

The invention discloses biomarkers of body fluids useful for the diagnostic, prognostic and/or for assessing the efficacy of a treatment or following the evolution of CMT disease.

### 1.1 CMT1A transgenic rat model and serum samples collection

The CMT transgenic rat model is a hemizygous PMP22 transgenic rat bearing three additional copies of mouse PMP22 gene show signs of demyelination in peripheral and cranial nerves (Sereda *et al.,* 1996; Grandis *et al.,* 2004). This CMT rat model is a good approximation of human CMT1A disease from a clinical point of view. Furthermore, the CMT rats already served as a model for an experimental CMT1A therapy (Meyer zu Hörste *et al.,* 2007).

Inventors have looked for small molecules showing differential levels in wild type and transgenic rats thus constituting relevant biomarkers for CMT disease.

Except otherwise specified, CMT1A model rats, four months old, are anaesthetized with Ketamine (Imalgene) 100 mg/kg, *ip.* Blood is collected by cardiac puncture in two different tubes:
- in one sterilized blood collection tube for coagulation; serum is collected and stored at -80°C
- in one EDTA RNAse free tube; after centrifugation (+4°C; 1260g; 10 min), plasma is stored at -80°C.

### 1.2 Quantitation methods

### • Free cholesterol

Cholesterol was firstly extracted from samples with heptanes. Free cholesterol was further analyzed using a method adapted from Dong *et al.* (2007): cholest-4-en-3,6-dione formed from the oxidation of non-esterified cholesterol by the Jones oxidation was measured by HPLC/UV analyses. Stigmasterol was used as an internal standard.

### • Sorbitol

Proteins are firstly precipitated with ethanol. Sorbitol is analyzed mainly according the Dionex N°20 technical note, using anion exchange chromatography coupled with electrochemical detector.

### • Metals

Quantitation of Iron and Zinc was performed by ICP/MS after mineralization of serum samples.

### • Arachidonic acid metabolites

Enzyme ImmunoAssays (EIA) kits from Cayman chemical were used to analyze:
Prostaglandin E₂ (ref. 514010)
Leukotriene B₄ (ref. 520111)
Thromboxane B₂ (ref. 519031)
6-keto Prostaglandine F_{1α} (ref. 515211)

### • Cyclic Adenosine MonoPhosphate

After precipitation of plasma proteins with ethanol, cAMP is analyzed with an EIA kit from Cayman chemical (ref 581001) according the manufacturer instructions.

### • Catecholamines

A solid phase extraction (SPE) was performed to concentrate and purify the samples. Adrenaline, dopamine, and serotoninserotonin were further analyzed by ion pair chromatography.

### • Amino Acids

Plasma proteins were precipitated with sulfosalicylic acid prior to analyzes. Derivatized amino acids quantitation was performed with a spectrophotometer after an automated cation exchange chromatography process.

### • Thyroid hormones

Prior to the precipitation of plasma proteins with methanol, an internal standard was added to samples. Triiothyronine (T3) and thyroxine (T4) were then quantified by an HPLC coupled to LC-MS/MS mainly according to Soukhova *et al* (2004).

### • Neurosteroids

CMT1A model rats, four months old, were decapitated and blood was collected in two different tubes:
- in one sterilized blood collection tube for coagulation; serum was collected and stored at - 80°C;
- in one EDTA RNAse free tube; after centrifugation (+4°C; 1260g; 10 min), plasma was stored at -80°C.

Prior to analysis, plasma proteins were precipitated and neurosteroids were then purified and concentrated by SPE. Neurosteroids were further chemically derivatized with either 2-hydrozino-1-methylpyridine (to lower the detection threshold (Higashi *et al.,* 2005) or picolinic acid (Yamashita *et al.,* 2007). According to derivatization method, internal standard is ²H testosterone or ²H 3α-androstanediol. HPLC analysis of Neurosteroids was coupled to a mass spectrometer. Searched neurosteroids and derivatives were aldosterone, pregnelonone sulfate, allopregnanolone, progesterone, 5α-dihydroprogesterone (DHP), 3α-androstanediol, testosterone, 5α dihydrotestosterone, DHEA and corticosterone.

### • Estrogens

Blood is collected as above. As for neurosteroids, estrone and estradiol are extracted from sample with ethyl acetate prior to derivatization with picolinic acid purification and a concentration and purification step by SPE. ²H-estrone and ²H-17β-estradiol are used as internal standards.

### 1.3 Results

Duplicate samples were analyzed for each biomarker. Statistical analysis (student test, bilateral, type 3) comparing WT rats versus CMT1A (transgenic) rats was performed. Results are summarized in the three tables below. These tables report the mean level of biomarkers which present a notable difference (*P*<0.2) between WT and CMT1A rats in male and female (table 1), in only male (table 2) and in only female (table 3).

The analysis of biomarkers has revealed that plasmatic free cholesterol level is significantly decreased in CMT1A male (P=0.05) and female rats (P=0.06) compared to WT rats. In females, our results displayed a significant decrease of alpha-aminobutyric acid (P=0.019), glutamine (P=0.025) and tyrosine (P=0.03) plasmatic levels versus controls.

Our results also show a significant decrease of the following biomarkers: alanine, cystine, glutamine, hydroxyproline, threonine, T4 thyroid hormone, citrulline, LTB4, adrenaline, and lysine; and a significant increase of the following biomarkers: tryptophan, testosterone, dopamine, serotonin, iron, methionine, and proline (Table 1, 2 and 3).

### 1.4 Fluid biological samples collection and quantification methods

Biomarkers of the invention can be easily quantified from other biological fluids. As an example quantitation from saliva sample are described by Kajalainen *et al.* (2007) for cholesterol, by Syrjänen *et al.* (1990) for glutamine and tyrosine. Likewise, those small molecules can be quantified in urine as described elsewhere for cholesterol (Cenedella *et al.,* 1981) and for amino acids (Venta *et al.,* 2001).

| **TABLE 1** | **WT** | | **TG** | | |
|---|---|---|---|---|---|
| **biomarkers** | **MEAN** | **s.e.m.** | **MEAN** | **s.e.m.** | ***P*** |
| **Free cholesterol (µg/ml)** | 144.82 | 4.18 | 118.45 | 3.96 | *0.0004* |
| **Alanine (µmol/l)** | 659.67 | 44.19 | 523.50 | 46.31 | *0.059* |
| **Cystine (µmol/l)** | 15.50 | 2.85 | 8.67 | 2.51 | *0.103* |
| **Glutamine (µmol/l)** | 792.00 | 60.46 | 694.50 | 18.11 | *0.174* |
| **Hydroxyproline (µmol/l)** | 54.00 | 4.41 | 41.33 | 6.31 | *0.135* |
| **Threonine (µmol/l)** | 295.00 | 20.30 | 258.17 | 15.76 | *0.184* |
| **Tryptophane (µmol/l)** | 79.83 | 4.76 | 93.00 | 3.94 | *0.060* |
| **Dopamine (µg/ml)** | 0.30 | 0.02 | 0.41 | 0.07 | *0.195* |
| **Serotonin (µg/ml)** | 112.50 | 22.82 | 406.05 | 133.26 | *0.079* |
| **T4 (µg/ml)** | 52.57 | 4.26 | 42.64 | 3.11 | *0.092* |
| **Iron (µg/ml)** | 4.77 | 0.45 | 7.03 | 1.24 | *0.134* |

| **TABLE 2** | **male** | | | | |
|---|---|---|---|---|---|
| | **WT** | | **TG** | | |
| **biomarkers** | **MEAN** | **s.e.m.** | **MEAN** | **s.e.m.** | ***P*** |
| **Free cholesterol (µg/ml)** | 146.11 | 7.80 | 120.74 | 6.78 | *0.050* |
| **Citrulline (µmol/l)** | 109.00 | 9.29 | 92.00 | 3.61 | *0.201* |
| **Methionine (µmol/l)** | 49.67 | 2.73 | 55.67 | 1.76 | *0.150* |
| **Proline (µmol/l)** | 224.67 | 19.54 | 265.00 | 14.50 | *0.179* |
| **Tryptophane (µmol/l)** | 84.33 | 5.24 | 99.00 | 4.93 | *0.111* |
| **Tyrosine (µmol/l)** | 87.00 | 5.13 | 98.67 | 3.93 | *0.150* |
| **Testosterone (µg/ml)** | 1.81 | 0.40 | 3.37 | 0.85 | *0.201* |
| **T4 (µg/ml)** | 59.23 | 3.21 | 48.84 | 2.00 | *0.063* |

| **TABLE 3** | **female** | | | | |
|---|---|---|---|---|---|
| | **WT** | | **TG** | | |
| **biomarkers** | **MEAN** | **s.e.m.** | **MEAN** | **s.e.m.** | ***P*** |
| **Free cholesterol (µg/ml)** | 143.54 | 4.41 | 116.16 | 4.88 | *0.006* |
| **LTB4 (pg/ml)** | 421.46 | 43.75 | 335.67 | 26.36 | *0.184* |
| **Alanine (µmol/l)** | 660.33 | 46.83 | 551.33 | 44.86 | *0.168* |
| **Alpha Amino Butyric acid (µmol/l)** | 13.33 | 1.33 | 6.67 | 0.88 | *0.019* |
| **Glutamine (µmol/l)** | 905.33 | 46.94 | 687.33 | 38.84 | *0.025* |
| **Hydroxyproline (µmol/l)** | 49.00 | 6.66 | 29.00 | 2.52 | *0.081* |
| **Lysine (µmol/l)** | 510.67 | 25.96 | 419.00 | 22.50 | *0.057* |
| **Tyrosine (µmol/l)** | 80.00 | 2.65 | 56.33 | 5.36 | *0.030* |
| **Adrenaline (µg/ml)** | 7.99 | 0.76 | 5.97 | 0.58 | *0.107* |

### II. Identification and Quantitation of other Cholesterol related biomarkers

### II.1 CMT1A transgenic rat model and serum samples collection

The CMT transgenic rat model and samples collection are the same as described above (see section I.1).

### 11.2 Cholesterol Quantitation methods

### • Total cholesterol

Total cholesterol has been determined by an enzymatic assay with ABX Pentra Cholesterol CP kit (Horiba). The cholesterol is consumed by cholesterol esterase and cholesterol oxidase in a color forming reaction where the color produced is proportional to the amount of the total cholesterol present in the sample.

### • LDL cholesterol

LDL cholesterol has been determined by an enzymatic assay with ABX Pentra LDL Direct CP kit (Horiba). The method is in a two reagents format and depends on the properties of the used detergents. The first detergent solubilizes all the non LDL lipoprotein particles. The cholesterol released is consumed by cholesterol esterase and cholesterol oxidase in a non-color forming reaction. the second detergent solubilizes the remaining LDL particles and a chromogenic coupler allows for color formation. The enzyme reaction in the presence of the coupler produces color that is specifically proportional to the amount of LDL cholesterol present in the sample.

### II.3 Results

Results presented in table 4 below were extracted from independent assays and analysed with a bilateral Student's t test comparing 20 WT rats versus 19 CMT1A (transgenic) rats.

**TABLE 4**

| | **WT** | | **TG** | | |
|---|---|---|---|---|---|
| **Biomarkers** | **MEAN** | **s.e.m** | **MEAN** | **s.e.m** | ***P*** |
| Total cholesterol | 1.81 | 0.07 | 1.77 | 0.05 | *0.66129* |
| LDL cholesterol | 0.26 | 0.02 | 0.21 | 0.01 | *0.03951* |

Our results show that LDL-cholesterol level is significantly decreased (P=0.039) in CMT1A rats (TG) compared to WT rats while total cholesterol level isn't significantly modified. LDL-cholesterol level is very easily quantifiable with commonly used detection kits.

### Correlation of biomarkers concentration with results from behavioral tests, histology, gene expression and electrophysiology

Motor performance and muscular strength, Sensory Nerve Action Potentials (SNAP), axonal diameter distribution and myelin sheath of fixed sciatic nerve, fiber content in fixed muscles and pmp22 mRNA expression were compared with biomarkers amount measured in biological fluids. The test used is a test of linear association between paired samples using Pearson's product moment correlation. It is a unilateral test and a significance threshold of 0.05 is applied on p-values.

Such analysis demonstrates that the levels of the biomarkers of the invention are correlated with some of above mentioned behavioral tests, histology, PMP22 gene expression and electrophysiology confirming thereby the significance of those biomarkers in CMT1A physiology and the pertinence of the use of these biomarkers in the diagnostic and follow-up of CMT1A.

### III. Identification of disease predictors from biomarkers of the invention

Statistical analysis of level of biomarkers of the invention obtained in above experiments shows that said biomarkers can also be used in different sets of grouped biomarkers to predict the presence of disease with a good score. Predictability scores are shown for some of possible sets comprising several of molecules identified herein as biomarkers for CMT disease (table 5).

Briefly, diagnostic of the disease was performed by applying a Linear-Discriminant-Analysis (LDA), commonly used in statistics, pattern recognition and machine learning to find a linear combination of features which characterize or separate two or more classes of objects. The LDA was implemented in R (http://www.r-project.org/).

The LDA algorithm was applied on several set of biomarkers selected on the basis of their correlation to the trait of interest (here transgenic *versus* wild-type). In order to properly assess the performances of each set of biomarkers, groups of rats were split into independent "training set" (75 % of rats) on which LDA was trained, and a "validation set" (25% of rats), on which the trained algorithm was validated. To be homogeneous, training and validation sets were made of equal proportions of transgenic/wild-type and male/female rats. Since the level of biomarkers differs between males and females, for a given set of biomarkers, LDA was trained and validated separately on males and females. Finally the trained LDA was used to classify each rat of the training and validation sets into "wild-type" and "transgenic", and the proportion of rats that were well-classified allowed assessing the performances of the algorithm. This procedure was reapplied iteratively in order to average the performances over all the possible samplings.

**TABLE 5**

| **Biomarkers** | **Training** | | | **Validation** | | |
|---|---|---|---|---|---|---|
| | **Male** | **Female** | **Male and Female** | **Male** | **Female** | **Male and Female** |
| Hydroxyproline and Alanine | 68% | 100% | 84% | 45% | 83% | 64% |
| Tryptophane and Hydroxyproline | 86% | 100% | 93% | 56% | 84% | 70% |
| T4 and Tryptophane and Hydroxyproline and Alanine | 92% | 100% | 96% | 60% | 89% | 75% |
| T4 and Hydroxyproline | 92% | 100% | 96% | 60% | 79% | 69% |
| T4 and Hydroxyproline and Alanine | 92% | 100% | 96% | 61% | 83% | 72% |
| Hydroxyproline and SerotoninSerotonin and Alanine | 81% | 100% | 90% | 61% | 84% | 72% |
| Hydroxyproline and SerotoninSerotonin | 74% | 100% | 87% | 61% | 72% | 67% |
| T4 and Tryptophane and Hydroxyproline | 94% | 100% | 97% | 61% | 89% | 75% |
| Tryptophane and Hydroxyproline and Alanine | 89% | 100% | 94% | 61% | 73% | 67% |
| Total of 20 biomarkers* | 100% | 100% | 100% | 62% | 72% | 67% |
| T4 and Tryptophane and Hydroxyproline and SerotoninSerotonin | 95% | 100% | 97% | 67% | 77% | 72% |
| Tryptophane and Hydroxyproline and SerotoninSerotonin and Alanine | 86% | 100% | 93% | 67% | 72% | 69% |
| T4 and Serotonin | 95% | 74% | 84% | 71% | 62% | 67% |
| Tryptophane and Hydroxyproline and Serotonin | 89% | 100% | 95% | 72% | 72% | 72% |
| T4 and Tryptophane and Serotonin and Alanine | 92% | 95% | 93% | 72% | 77% | 75% |
| T4 and Tryptophane | 95% | 97% | 96% | 72% | 77% | 75% |
| T4 and Hydroxyproline and Serotonin and Alanine | 85% | 95% | 90% | 72% | 83% | 78% |
| T4 and Tryptophane and Alanine | 94% | 97% | 96% | 72% | 78% | 75% |
| T4 and Hydroxyproline and Serotonin | 91% | 100% | 96% | 73% | 71% | 72% |
| T4 and Alanine | 89% | 83% | 86% | 73% | 73% | 73% |
| T4 and Tryptophane and Hydroxyproline and Serotonin and Alanine | 94% | 100% | 97% | 73% | 83% | 78% |
| Serotonin and Alanine | 78% | 78% | 78% | 73% | 71% | 72% |
| T4 and Serotonin and Alanine | 86% | 78% | 82% | 73% | 71% | 72% |
| T4 and Tryptophane and Serotonin | 94% | 97% | 96% | 73% | 77% | 75% |
| Tryptophane and Alanine | 92% | 83% | 87% | 77% | 61% | 69% |
| Free-cholesterol and Hydroxyproline and Serotonin and Alanine | 100% | 100% | 100% | 78% | 78% | 78% |
| Free-cholesterol and Tryptophane and Hydroxyproline and Alanine | 100% | 100% | 100% | 78% | 84% | 81% |
| Free-cholesterol and Tryptophane and Hydroxyproline and Serotonin and Alanine | 100% | 100% | 100% | 78% | 72% | 75% |
| Free-cholesterol and Hydroxyproline and Alanine | 100% | 100% | 100% | 78% | 88% | 83% |
| Tryptophane and Serotonin | 86% | 82% | 84% | 78% | 66% | 72% |
| Free-cholesterol and Tryptophane and Hydroxyproline | 100% | 100% | 100% | 83% | 83% | 83% |
| Free-cholesterol and Tryptophane and Alanine | 100% | 100% | 100% | 84% | 72% | 78% |
| Tryptophane and Serotonin and Alanine | 83% | 84% | 83% | 84% | 72% | 78% |
| Free-cholesterol and Tryptophane and Serotonin and Alanine | 100% | 100% | 100% | 84% | 72% | 78% |
| Free-cholesterol and Hydroxyproline and Serotonin | 100% | 100% | 100% | 84% | 77% | 80% |
| Free-cholesterol and Tryptophane and Hydroxyproline and Serotonin | 100% | 100% | 100% | 84% | 73% | 79% |
| Free-cholesterol and Serotonin and Alanine | 100% | 100% | 100% | 84% | 79% | 81% |
| Free-cholesterol and T4 and Serotonin and Alanine | 100% | 100% | 100% | 88% | 78% | 83% |
| Free-cholesterol and T4 and Tryptophane and Alanine | 100% | 100% | 100% | 89% | 88% | 88% |
| Free-cholesterol and T4 and Hydroxyproline and Alanine | 100% | 100% | 100% | 89% | 90% | 89% |
| Free-cholesterol and T4 and Tryptophane and Serotonin and Alanine | 100% | 100% | 100% | 89% | 77% | 83% |
| Free-cholesterol and T4 and Tryptophane and Hydroxyproline and Serotonin and Alanine | 100% | 100% | 100% | 89% | 78% | 84% |
| Free-cholesterol and T4 and Tryptophane and Hydroxyproline and Alanine | 100% | 100% | 100% | 89% | 88% | 89% |
| Free-cholesterol and T4 and Hydroxyproline and Serotonin and Alanine | 100% | 100% | 100% | 89% | 78% | 84% |
| Free-cholesterol and T4 and Tryptophane and Hydroxyproline | 100% | 100% | 100% | 94% | 94% | 94% |
| Free-cholesterol and T4 and Hydroxyproline and Serotonin | 100% | 100% | 100% | 94% | 77% | 86% |
| Free-cholesterol and T4 and Tryptophane and Serotonin | 100% | 100% | 100% | 94% | 79% | 87% |
| Free-cholesterol and T4 and Alanine | 100% | 100% | 100% | 94% | 83% | 89% |
| Free-cholesterol and T4 and Tryptophane and Hydroxyproline and Serotonin | 100% | 100% | 100% | 94% | 77% | 86% |
| Free-cholesterol and Hydroxyproline | 100% | 100% | 100% | 94% | 94% | 94% |
| Free-cholesterol and Tryptophane and Serotonin | 100% | 100% | 100% | 95% | 67% | 81% |
| Free-cholesterol and Alanine | 100% | 100% | 100% | 95% | 89% | 92% |
| Free-cholesterol and T4 and Tryptophane | 100% | 100% | 100% | 95% | 89% | 92% |
| Free-cholesterol and Tryptophane | 100% | 100% | 100% | 95% | 78% | 86% |
| Free-cholesterol and T4 and Serotonin | 100% | 100% | 100% | 100% | 78% | 89% |
| Free-cholesterol and Serotonin | 100% | 100% | 100% | 100% | 79% | 89% |
| Free-cholesterol and T4 and Hydroxyproline | 100% | 100% | 100% | 100% | 95% | 97% |
| Free-cholesterol and T4 | 100% | 100% | 100% | 100% | 95% | 97% |

| | | | | | | |
|---|---|---|---|---|---|---|
| * Total of 20 biomarkers: Free Cholesterol, T4, Tryptophane, Hydroxyproline, Serotonin, Alanine, alpha-AminoButyricAcid, Citrulline, Cystine, Glutamine, Lysine, Methionine, Proline, Threonine, Tyrosine, Testosterone, Iron, LTB4, Adrenaline, Dopamine. | | | | | | |

### IV. Biomarkers analysis in CMT1A patients

An analysis of blood biomarkers was performed as a secondary objective of a double-blind, randomized, placebo-controlled Phase 2 study (ClinicalTrials.gov Identifier: NCT01401257) which primary objective is to assess the clinical and laboratory safety and tolerability of three doses of a mix of baclofen, naltrexone and sorbitol (MIX), a candidate treatment administered orally for 12 months to CMT1A patients versus placebo.

Patients aged 18-65 years were included with CMT1A diagnosis based on clinical examination and confirmation by genotyping (duplication in 17p11.2), weakness in at least foot dorsiflexion, and a Charcot-Marie-Tooth Neuropathy Score (CMTNS) ≤ 20, i.e. a mild to moderate severity. Eligible patients were randomly assigned in a 1:1:1:1 ratio to receive daily for one year Placebo, Low dose (LD = 0.6 mg baclofen, 0.07 mg naltrexone and 21 mg sorbitol), Intermediate dose (ID = 1.2 mg baclofen, 0.14 mg naltrexone and 42 mg sorbitol) or High dose (HD = 6 mg baclofen, 0.7 mg naltrexone and 210 mg sorbitol) of the mix.

The results regarding the efficacy of the treatment of this Phase 2 study have been published (Attarian *et al.,* 2014). Patients treated with the highest dose show consistent evidence of improvement beyond stabilization of the disease, after one year of treatment.

### IV.1 Biomarkers collection

Blood samples were taken both at randomization and after 3 months of treatment with the mix (3 doses tested) or placebo. Following sample collection in lithium-heparin, tubes were centrifuged for 10 min at 1300 g at room temperature, and plasma samples were stored at -80°C until analysis.

Plasma concentrations were determined using HPLC coupled with Mass Spectrometry detection (LC-MS/MS) after protein precipitation for L-alanine and L-tryptophan and Liquid/Liquid extraction for Free Cholesterol.

The Lower Limits of Quantification (LLOQ) were 20 µg/mL, 10 µg/mL, 5 µg/mL for free cholesterol, L-alanine, L-tryptophan respectively. The results are expressed as µg/mL for L-alanine, free cholesterol and L-tryptophan.

### IV.2 Efficacy endpoints

The clinical endpoints considered in the trial are listed in table 6:

**Table 6**

| **Endpoints** | **Improvement (direction of variation for an improvement)** | ***Reference*/*comments*** |
|---|---|---|
| **Clinical scales** | | |
| **CMTNS** | ↓ | Shy et al. (2005) |
| **ONLS** | ↓ | Graham and Hughes (2006) |

| **Functional measures** | | |
|---|---|---|
| **6MWT (m)** | ↑ | Guyatt et al. (1985) |
| **9HPT (s)** | ↓ | Hogrel et al. (2007) (*non dominant hand considered*) |
| **Ankle Dorsiflexion (Nm)** | ↑ | Hogrel et al.(2007) (*mean of left and right side considered*) |
| **Grip (kg)** | ↑ | Hogrel et al. (2007) (*non dominant hand considered*) |
| Electrophysiological parameters | | |
| **CMAP (mV)** | ↑ | *measured from the mean sensory* |
| **MCV (m/s)** | ↑ | *responses of the median and ulnar nerves* (*non-dominant side*) |

Compound muscle action potential (CMAP) is an electromyography investigation which represents the summation of a group of almost simultaneous action potentials from several muscle fibers in the same area which are evoked by stimulation of the motor nerve. Patients with impaired peripheral nerves show a decreased CMAP.

Motor conduction velocity (MCV) is the speed at which an electrical stimulation of a nerve propagates down to a muscle supplied by this nerve. Patients suffering from motor neuropathies display reduced speeds.

### IV.3 Statistical analysis

All analyses were performed on the Full Analysis Set (all randomized patients) using *R* version 3.1.2 (http://cran.r-project.org). Considering the exploratory nature of the study, statistical tests were conducted at a two-sided 5% level. Correlations were assessed using a Spearman's rank test. When specified, correlations were assessed based on data adjusted on Gender, Age and Centre with a linear model. Comparisons of two groups were performed using a Welch t-test; comparisons of more than two groups were performed using an Analysis of Variance (ANOVA).

### Effect of treatment analysis

Differences at 3 months between patients under baclofen, naltrexone and sorbitol mix treatment and patients under placebo were assessed by Analysis of Covariance (ANCOVA) adjusted on baseline values and including also gender, age and clinical centre as fixed covariates. The significance of the treatment effect on the combination of two biomarkers was assessed through the O'Brien's OLS test.

### Identification of responders

Biomarker levels at baseline between non-deteriorated patients (e.g. exhibiting improved or stabilized symptoms) and deteriorated (exhibiting a worsened condition) at 12 months following the approach of Attarian et al. (2014) on efficacy endpoints have been compared.

### IV. 4 Results

### Correlation between disease state or evolution and biomarkers

A baseline correlation analysis has been performed between efficacy endpoints and biomarkers by adjusting for gender, age and clinical centre in order to take into account any variation not related to the disease state.

The level of tryptophan is found to correlate significantly with all efficacy endpoints (table 7). This correlation is positive with endpoints for which an increase means improvement and negative with endpoints for which a decrease means improvement. Consequently, higher tryptophan levels are found associated with less severe disease profiles.

Alanine also shows correlation with some of the endpoints (table 7).

Noteworthy, a significant and positive correlation between the two biomarkers (R = 0.44, *p* = 7e-05) is observed, which confirms the correlation to efficacy endpoints observed for both tryptophan and alanine.

**Table 7**

| **Endpoints** | **Improvement** (direction of variation for an improvement) | **Spearman's coefficient correlation** *(p<0.05)* | |
|---|---|---|---|
| | | **L-Trp** | **L-Ala** |
| **Clinical scales** | | | |
| **CMTNS** | ↓ | **-0.27** | **ns** |
| **ONLS** | ↓ | **-0.29** | **ns** |

| **Functional measures** | | | |
|---|---|---|---|
| **6MWT (m)** | ↑ | **+0.4** | **+0.3** |
| **9HPT (s)** | ↓ | **-0.25** | **ns** |
| **Ankle Dorsiflexion (Nm)** | ↑ | **+0.36** | **ns** |
| **Grip (kg)** | ↑ | **+0.26** | **ns** |

| **Electrophysiological parameters** | | | |
|---|---|---|---|
| **CMAP (mV)** | ↑ | **+0.34** | **+0.36** |
| **MCV (m/s)** | ↑ | **+0.28** | **+0.24** |

Of note, the efficacy endpoints correlate well and significantly with each other and in a coherent way with regard to the severity of the disease. Then, even if moderate, due to the multidimensional nature of the disease, efficacy endpoints and also variation of tryptophan and Alanine bear a predictable relationship to the overall disease severity.

Hence, tryptophan and/or alanine level(s) can be used to assess the evolution and the severity of CMT1A in the patient population.

### Biomarkers levels as early markers of the efficacy of a treatment.

The effect of 3 months treatment with baclofen, naltrexone and sorbitol on the biomarkers levels was assessed (table 8). A significant increase in tryptophan (p = 0.018) and alanine (p = 0.04) in patients treated for 3 months with the mix (1.56 ± 2.98 µg/mL and 3.05 ± 8.16 µg/mL respectively) compared to placebo (0.43 ± 1.45 µg/mL and 0.75 ± 6.75 µg/mL respectively) is found. When these two markers were considered jointly, the significance of the effect to treatment was even greater (p = 0.0086). Noteworthy, after 3 months of treatment no symptomatic change is evidenced in the treated population, because of the slow and progressive nature of the disease. Biomarkers of the invention thus provide efficient tools to determine the response to a treatment.

**Table 8**

| | **Placebo** **(*n* = 19)** | | **MIX HD** **(n = 19)** | | **MIX HD vs Placebo** | |
|---|---|---|---|---|---|---|
| **Biomarker** | **Baseline** | **Change from Baseline** | **Baseline** | **Change from Baseline** | **Estimate** | ***p*** |
| **Alanine (µg/ml)** | 30.17 (6.80) | 0.75 (6.75) | 32.77 (11.55) | 3.05 (8.16) | 4.0 (0.19;7.87) | 0.04* |
| **Tryptophan (µg/ml)** | 10.26 (2.19) | 0.43 (1.45) | 10.69 (1.75) | 1.56 (2.98) | 1.6 (0.29;2.86) | 0.018* |

| | | | | | | |
|---|---|---|---|---|---|---|
| MIX: mix of baclofen, naltrexone and sorbitol; HD: High Dose of MIX (6 mg baclofen, 0.7 mg naltrexone and 210 mg sorbitol). *data are mean changes from baseline (s.d.); Estimate : differences of change from baseline adjusted on Gender, Age and Centre, least squares mean (95% confidence interval). (ANCOVA, *p < 0.05). | | | | | | |

### Free cholesterol levels to discriminate responders from non-responders to treatments of CMT

Among patients treated with baclofen, naltrexone and sorbitol, those for which a worsen in their condition (non-responders) was observed after one year treatment were found to have a significantly higher plasma concentration in free cholesterol than the responders (p = 0.034) at the beginning of the study (Fig 1). Hence biomarkers of the invention can be used as a predictor of the response or responsiveness to baclofen, naltrexone and sorbitol combinatory treatment.

### BIBLIOGRAPHY

Attarian, S. et al. An Exploratory Randomised Double-Blind and Placebo-Controlled Phase 2 Study of a Combination of Baclofen, Naltrexone and Sorbitol (PXT3003) in Patients with Charcot-Marie-Tooth Disease Type 1A. Orphanet Journal of Rare Diseases (2014);9: 1-15.
Berger, P. et al. Schwann cells and the pathogenesis of inherited motor and sensory neuropathies (Charcot-Marie-Tooth disease). Glia. 2006; 54(4):243-257.
Burns, J. et al. Ascorbic acid for Charcot-Marie-Tooth disease type 1A in children: a randomised, double-blind, placebo-controlled, safety and efficacy trial. Lancet Neurol. 2009; 8(6):537-544.
Cenedella, R.J. et al. Studies on the source of urinary cholesterol in the normal human male. The Journal of Lipid Research. 1981; 22:122-130.
Chumakov et al. Polytherapy with a combination of three repurposed drugs (PXT3003) down-regulates Pmp22 overexpression and improves myelination, axonal and functional parameters in models of CMT1A neuropathy. Orphanet Journal of Rare Diseases (2014); vol. 9, no. 1: 201.
Dong, J. et al. Jones oxidation and high performance liquid chromatographic analysis of cholesterol in biological samples J. Chromatogr. B (2007);858:239-246.
Graham, R.C., and Hughes R.A.C. A Modified Peripheral Neuropathy Scale: The Overall Neuropathy Limitations Scale. Journal of Neurology, Neurosurgery, and Psychiatry (2006);77 (8): 973-76.
Grandis, M. et al. Early abnormalities in sciatic nerve function and structure in a rat model of Charcot-Marie-Tooth type 1A disease. Exp Neurol. (2004);190(1):213-23.
Grandis, M. & Shy, M.E. Current Therapy for Charcot-Marie-Tooth Disease. Curr Treat Options Neurol. 2005; 7(1):23-31.
Guyatt, G.H. et al. The 6-Minute Walk: A New Measure of Exercise Capacity in Patients with Chronic Heart Failure. Canadian Medical Association Journal. (1985) ;132 (8): 919-23.
Higashi, T. et al. 2-Hydrazino-1-methylpyridine: a highly sensitive derivatization reagent for oxosteroids in liquid chromatography-electrospray ionization-mass spectrometry. samples J. Chromatogr. B (2005);825:214-222.
Hogrel, J.Y. et al. Development of a French Isometric Strength Normative Database for Adults Using Quantitative Muscle Testing. Archives of Physical Medicine and Rehabilitation (2007) 88 (10): 1289-97. doi:10.1016/j.apmr.2007.07.011.
Kapur, S. et al. Anesthetic management of a parturient with neurofibromatosis 1 and Charcot-Marie-Tooth disease. J Clin Anesth. 2007;19(5):405-406.
Karjalainen, S. et al. Salivary cholesterol of healthy adults in relation to serum cholesterol concentration and oral health. J Dent Res. 1997;76(10):1637-1643.
Katona, I. et al. PMP22 expression in dermal nerve myelin from patients with CMT1A. Brain. 2009; 132(Pt 7):1734-1740.
Li, J. et al. Skin biopsies in myelin-related neuropathies: bringing molecular pathology to the bedside. Brain. 2005;128(Pt 5):1168-1177.
Meyer zu Horste, G. et al. Antiprogesterone therapy uncouples axonal loss from demyelination in a transgenic rat model of CMT1A neuropathy. Ann Neurol. (2007); 61(1):61-72.
Nave, K.A. et al., Mechanisms of disease: inherited demyelinating neuropathies. Nat Clin Pract Neurol. 2007; 3(8): 453-464.
Niebrój-Dobosz, I. et al. Serum lipids in various polyneuropathies. Neurol Neurochir Pol. 197711(4):421-426
Niemann, A. et al. Pathomechanisms of mutant proteins in Charcot-Marie-Tooth disease. Neuromolecular Med. 2006; 8(1-2):217-242.
Passage, E. et al. Ascorbic acid treatment corrects the phenotype of a mouse model of CMT disease. Nature Med. 2004; 10(4): 396-401.
Patroclo, C.B. et al. Autosomal dominant HMSN with proximal involvement: new Brazilian cases. Arquivos de Neuro-Psiquiatria. 2008; 67(3B):892-896
Sahenk, Z. et al. NT-3 promotes nerve regeneration and sensory improvement in CMT1A mouse models and in patients. Neurology. 2005; 65(5):681-689.
Sereda, M. et al. A transgenic rat model of Charcot-Marie-Tooth disease. Neuron. (1996); 16(5): 1049-60.
Sereda, M.W. et al. Therapeutic administration of progesterone antagonist in a model of Charcot-Marie-Tooth disease (CMT-1A). Nat Med 2003; 9: 1533-1537.
Shy, M.E. et al. Reliability and Validity of the CMT Neuropathy Score as a Measure of Disability. Neurology 2005;64 (7): 1209-14.
Suter, U. & Scherer, S.S. Disease mechanisms in inherited neuropathies. Nat. Rev. Neurosci. 2003; 4: 714-726.
Soukhova, N. et al. Isotope dilution tandem mass spectrometric method for T4/T3. Clin Chim Acta. (2004);343(1-2): 185-90.
Syrjänen, S.M. et al. Free amino acid levels in oral fluids of normal subjects and patients with periodontal disease. Arch Oral Biol. 1990;35(3):189-193.
Venta, R. et al. Year-Long Validation Study and Reference Values for Urinary Amino Acids Using a Reversed-Phase HPLC Method. Clinical Chemistry. 2001; 47: 575-583
Weiner, D.S. et al. The Akron dome midfoot osteotomy as a salvage procedure for the treatment of rigid pes cavus: a retrospective review. J Pediatr Orthop. 2008; 28(1):68-80.
Yamashita, K. et al. Highly sensitive determination of estrone and estradiol in human serum by liquid chromatography-electrospray ionization tandem mass spectrometry. Steroids (2007);72:819-827.
Yao, J.K. et al. Lipid abnormalities in hereditary neuropathystar: Part 2. Serum phospholipids. Journal of the Neurological Sciences. 1978; 36(2):225-236.
Young, P. et al. Treatment for Charcot-Marie-Tooth disease. Cochrane Database Syst Rev. 2008; (1): CD006052.

## Claims

1. An *in vitro* method for assessing the evolution of CMT (Charcot-Marie-Tooth) disease in an individual having CMT and undergoing a treatment for CMT, the method comprising:
i) determining the levels of alanine and tryptophan in a biological sample from said individual, and
ii) comparing the levels of alanine and tryptophan obtained in i) to the levels of alanine and tryptophan, determined previously in the same individual before the beginning of the treatment, or at an early stage of said treatment,
wherein the time interval between the two measures of the levels of alanine and tryptophan from steps i) and ii) is at least 2 months, and wherein a relative increase in the levels of alanine and tryptophan is indicative of an improvement or stabilization of CMT disease in said individual.

2. The method of claim 1, wherein the time interval between the two measures of alanine and tryptophan levels from steps i) and ii) is at least 3 or 4 months.

3. The method of claim 1, wherein the treatment is continued in an individual in whom an increase of alanine and tryptophan levels is determined.

4. The method of claim 1, wherein said treatment comprises administering baclofen, naltrexone and sorbitol, or salts thereof, to said individual.

5. The method of claim 1, wherein said CMT disease is CMT1A.

6. The method of claim 1, wherein said biological sample is a fluid biological sample, preferably blood or plasma.

## Patentansprüche

1. Ein *in-vitro*-Verfahren zur Beurteilung der Entwicklung der CMT (Charcot-Marie-Tooth) -Krankheit bei einer Person mit CMT und unter CMT-Behandlung, wobei das Verfahren umfasst:
i) Bestimmen der Alanin- und Tryptophan-Spiegel in einer biologischen Probe von diesem Individuum und
ii) Vergleichen der in i) erhaltenen Alanin- und Tryptophan-Spiegel mit den Alanin- und Tryptophan-Spiegeln, die zuvor bei derselben Person vor Beginn der Behandlung oder in einem frühen Stadium der Behandlung bestimmt wurden;
wobei das Zeitintervall zwischen den beiden Messungen der Alanin- und Tryptophan-Spiegel aus den Schritten i) und ii) mindestens 2 Monate beträgt und wobei ein relativer Anstieg der Alanin- und Tryptophan-Spiegel eine Verbesserung oder Stabilisierung der CMT-Krankheit in besagter Person anzeigt.

2. Verfahren nach Anspruch 1, wobei das Zeitintervall zwischen den beiden Messungen der Alanin- und Tryptophan-Spiegel aus den Schritten i) und ii) mindestens 3 oder 4 Monate beträgt.

3. Verfahren nach Anspruch 1, wobei die Behandlung bei einer Person fortgesetzt wird, bei der ein Anstieg der Alanin- und Tryptophan-Spiegel bestimmt wird.

4. Verfahren nach Anspruch 1, wobei die Behandlung die Verabreichung von Baclofen, Naltrexon und Sorbit oder Salzen davon an dieses Individuum umfasst.

5. Verfahren nach Anspruch 1, wobei diese CMT-Krankheit CMT1A ist.

6. Verfahren nach Anspruch 1, wobei diese biologische Probe eine flüssige biologische Probe ist, vorzugsweise Blut oder Plasma.

## Revendications

1. Méthode pour évaluer *in vitro* l'évolution de la maladie CMT (Charcot-Marie-Tooth) chez un individu atteint de la CMT et soumis à un traitement pour la CMT, la méthode comprenant les étapes suivantes:
i) déterminer les niveaux d'alanine et de tryptophane dans un échantillon biologique dudit individu, et
ii) comparer les niveaux d'alanine et de tryptophane obtenus à l'étape i) aux niveaux d'alanine et de tryptophane, déterminés précédemment chez le même individu avant le début du traitement, ou à un stade précoce dudit traitement,
dans laquelle l'intervalle de temps entre les deux mesures des niveaux d'alanine et de tryptophane des étapes i) et ii) est d'au moins 2 mois, et dans laquelle une augmentation relative des niveaux d'alanine et de tryptophane est indicative d'une amélioration ou d'une stabilisation de la maladie CMT chez ledit individu.

2. Méthode de la revendication 1, dans laquelle l'intervalle de temps entre les deux mesures des niveaux d'alanine et de tryptophane des étapes i) et ii) est d'au moins 3 ou 4 mois.

3. Méthode de la revendication 1, dans laquelle le traitement est poursuivi chez un individu chez lequel une augmentation des niveaux d'alanine et de tryptophane est déterminée.

4. Méthode de la revendication 1, dans laquelle ledit traitement comprend l'administration de baclofène, de naltrexone et de sorbitol, ou leurs sels, audit individu.

5. Méthode de la revendication 1, dans laquelle ladite maladie CMT est la CMT1A.

6. Méthode de la revendication 1, dans laquelle ledit échantillon biologique est un échantillon biologique liquide, de préférence du sang ou du plasma.
